# EUROPÄISCHE PATENTANMELDUNG

(11) **EP 0 742 286 A2**
(43) Veröffentlichungstag der Anmeldung: **13.11.1996**
(21) Anmeldenummer: 96107141.2
(22) Anmeldetag: 07.05.1996
(51) Int. Cl.: C12Q 1/68

(54) **Verfahren zum quantitativen Nachweis von Nukleinsäuren**

(30) Priorität: 08.05.1995 DE 19516196
(71) Anmelder: BOEHRINGER MANNHEIM GMBH, 68298 Mannheim (DE)
(72) Erfinder: Doppler, Clemens, Dr., 68782 Brühl (DE); Fritton, Hans-Peter, Dr., 69509 Mörlenbach (DE); Hinzpeter, Mathias, Dr., 80689 München (DE); Leying, Hermann, Dr., 83673 Bichl (DE); Wittor, Heiko, 82327 Tutzing (DE)

(57) **Zusammenfassung**

Die Erfindung beschreibt ein Verfahren zur quantitativen Bestimmung von spezifischen Polynukleotidsequenzen, das im wesentlichen dadurch gekennzeichnet ist, daß eine aus einer Mischung, wie z.B. einer biologischen Probe isolierte einzelsträngige Nukleinsäure, insbesondere mRNA, in Lösung mit einer im wesentlichen zur zu bestimmenden Sequenz komplementären Polynukleotid-Sequenz hybridisiert, anschließend an eine Festphase immobilisiert und die Menge an gebundenem Hybrid bestimmt wird. Als besonders geeignet hat sich erwiesen, wenn die Bindung an die beschichtete Festplatte mittels einer spezifisch bindbaren chemischen Gruppe über eine Linkerfunktion gekoppelt an die zu bestimmende Sequenz oder die Polynukleotid-Sonden-Sequenz erfolgt.

## Beschreibung

Die Erfindung beschreibt ein Verfahren zur quantitativen Bestimmung von spezifischen Polynukleotid-Sequenzen, das im wesentlichen dadurch gekennzeichnet ist, daß eine aus einer Mischung, wie z.B. einer biologischen Probe isolierte einzelsträngige Nukleinsäure, insbesondere mRNA, in Lösung mit einer im wesentlichen zur zu bestimmenden Sequenz komplementären Polynukleotid-Sequenz hybridisiert, anschließend an eine Festphase immobilisiert und die Menge an gebundenem Hybrid bestimmt wird. Als besonders geeignet hat sich erwiesen, wenn die Bindung an die beschichtete Festphase mittels einer spezifisch bindbaren chemischen Gruppe gekoppelt an die zu bestimmende Sequenz oder die Polynukleotid-Sonden-Sequenz erfolgt.

Eine Reihe von Verfahren zum Nachweis von Nukleinsäuren sind heute bekannt. Diese beruhen in der Regel auf dem Prinzip der Hybridisierung, wobei in den meisten Fällen zunächst die Immobilisierung der zu bestimmenden Sequenz an die Festphase erfolgt und anschließend eine markierte Nukleinsäure-Probe zugegeben wird. Das Verfahren ist jedoch zeitaufwendig und für den ungeübten Praktiker nicht ohne weiteres mit Erfolg durchzuführen. Dies gilt insbesondere deshalb, da eine Hybridisierung an der Festphase wenig effizient verläuft.

Alternativ kann die Bestimmung von Nukleinsäuren über die in situ-Markierung der Proben-Nukleinsäure und die Fixierung an die Festphase, vermittelt über eine sequenzspezifische Nukleotidsequenz-Probe, erfolgen. In einem weiteren Verfahren werden zwei sequenzspezifische Probes für die zu bestimmende Nukleinsäure herangezogen. Sowohl die in situ-Markierung, als auch die Hybridisierung mit zwei Probes an der Festphase verlaufen oft nicht reproduzierbar, d.h. sind schwer oder nur mit großer Ungenauigkeit quantifizierbar, sind dazu experimentell aufwendig und somit für die Routine der klinischen Diagnostik wenig geeignet. Entsprechende Verfahren bzw. Varianten sind als Northern-Blot-Verfahren, Nuclease-Protection-Assay und quantitative RT-PCR-Verfahren bekannt und gehören heute zu den Standardmethoden zur Quantifizierung von Nukleinsäuren (T. Maniatis, Molecular Cloning: A Laboratory Manual, 2nd ed. (1989); R. E. Farell, RNA Modologies: A Laboratory Guide for Isolation and Characterization, Academic Press; J. W. Larrick, Trends Biotechnol. 10, 146-152 (1992); E. S. Kawasaki, A Guide to Methods and Applications (eds. Innis, M.A. et al) Academic Press).

Außerdem ist der Nachweis von Nukleinsäuren, insbesondere von mRNA im sogenannten Mikrotiterplatten-Verfahren bekannt, wobei die Hybridisierungsreaktion in Lösung erfolgt. In der Regel erfolgt dabei die Hybridisierung mit einer Biotin-markierten cDNA-Probe. Die Nukleinsäurehybride werden anschließend über die Biotin-markierung immobilisiert und mit einem Antikörper, der spezifisch DNA/RNA-Hybride bindet in einem herkömmlichen ELISA-Verfahren detektiert (C. O. Yehle et al., Mol. Cell. Probes 1, 177-193 (1987); F. Countlee et al, J. Biol. Chem. 256, 11601-11604 (1990); EP 0 336 454). Ferner ist es möglich anstatt eines Antikörpers ein geeignetes Detektionsprobe zu verwenden (sogen. Sandwichhybridisierung, EP 0 192 168).

Nachteilig bei Verfahren dieser Art ist jedoch zum einen, daß lediglich DNA als Fangprobe verwendet werden kann und zwar bedingt durch die Tatsache, daß der Nachweis über DNA/RNA-spezifische Antikörper erfolgt. Zum anderen ist das System unter Verwendung von herkömmlichen chromogenen Substraten nur wenig sensitiv. Darüber hinaus hat sich bei der Verwendung von photoreaktiven Substanzen als Markierungsreagenz für Nukleinsäureprobes gezeigt, daß die Sensitivität unzureichend ist und die Handhabbarkeit entsprechender Bestimmungsverfahren zu wünschen übrig läßt (EP 0 237 833).

Auch ein erst kürzlich publiziertes Verfahren, bei dem RNA zunächst mit einer bereits im Mikrotiterplatten-well immobilisierten Fangprobe hybridisiert und anschließend mit einem fluoreszierenden interkalierendem Agenz markiert und detektiert wird (T. Okamoto et al, Anal. Biochem. 221, 202-204 (1994)), überkommt die Nachteile nur zum Teil. In Abhängigkeit von der Länge der Fangprobe führt dieses Verfahren zu hohem Hintergrundsignalen, da nicht nur die eigentlich nachzuweisende RNA, sondern auch die immobilisierte Fangprobe markiert wird.

Aufgabe der zugrundeliegenden Erfindung ist daher, ein Verfahren zu Bestimmung einer spezifischen Polynukleotidsequenz zur Verfügung zu stellen, durch das die Nachteile der im Stand der Technik beschriebenen Verfahren überwunden werden, d.h. das insbesondere leicht durchführbar und automatisierbar ist und mit dem Nukleinsäuren quantitativ erfaßt werden können.

Gelöst wird die Aufgabe durch ein Verfahren zur Bestimmung einer spezifischen Polynukleotidsequenz in einer Probenmischung, welches folgende Schritte umfaßt: Die Nukleinsäuren, insbesondere solche mit Poly-dA-Sequenzen (mRNA) werden isoliert und, soweit noch erforderlich, in einzelsträngige Nukleinsäuren überführt.

Anschließend erfolgt die Markierung der zu bestimmenden einzelsträngigen Nukleinsäure mit einer chemischen Gruppe, die vorzugsweise über eine Linkerfunktion an das Nukleinsäuremolekül, vorteilhafterweise in nicht-kovalenter gebunden ist bzw. assoziiert ist. Als chemische Gruppen sind solche geeignet, durch die entweder die Bindung an die Festphase vermittelt wird, oder die in einem direkten oder indirektem Verfahren detektierbar sind. Als immobilisierbare chemische Gruppen haben sich spezifisch bindbare Liganden wie z.B. Biotin oder Haptene wie z.B. Digoxigenin als vorteilhaft erwiesen.

Die markierte Nukleinsäure wird dann mit einer Polynukleotid-Sonde, die mindestens eine einzelsträngige Basensequenz umfaßt, die im wesentlichen komplementär zu der zu bestimmenden Sequenz ist, in Lösung unter Bedingungen, die für eine Hybridisierung zwischen der zu bestimmenden Sequenz und der komplementären Sondensequenz günstig sind, hybridisiert. Die Sondensequenz ist mit einer zweiten, von der ersten unterschiedlichen chemischen Gruppe markiert. Hier kommen prinzipiell wie oben immobilisierbare oder bestimmbare chemische Gruppen in Betracht mit der Maßgabe, daß die erste und zweite Gruppe nicht identisch sein dürfen.

Das zweifach markierte Nukleinsäurehybrid wird über eine Markierungsgruppe an die Festphase gebunden, und über die andere wird die Menge an gebundenem Hybrid und somit die aus einem bestimmten Volumen isolierte Nukleinsäure quantifiziert.

Insbesondere als vorteilhaft hat sich das erfindungsgemäße Verfahren für die Quantifizierung von Poly-dA-Sequenzen beinhaltende Nukleinsäuren wie mRNA erwiesen. Zur Hybridisierung können alle Arten von Proben verwendet werden, insbesondere anti-sense RNA und sogenannte "Peptide Nucleic Acid" (PNA). Dies ist von Bedeutung, da die Hybridisierung zwischen PNA- und RNA-Molekülen effizienter erfolgt als zwischen reinem RNA-Molekülen und diese wiederum effizienter hybridisieren als DNA- und RNA-Moleküle.

Der Einbau einer großen Anzahl von Markierungen in die nachzuweisenden RNA oder in die zum Nachweis benutzte DNA erlaubt eine Erhöhung des Meßsignals und damit insbesondere auch den chromogenen Nachweis von spezifischer mRNA, was bei den vorbekannten Verfahren nur bedingt möglich ist.

Ein weiterer Vorteil des erfindungsgemäßen Verfahrens besteht darin, daß die zur Immobilisierung eingesetzte Probe nicht markiert wird. Dies führt zu einer erheblichen Reduktion des Hintergrundes.

Zudem ist bei dem erfindungsgemäßen Verfahren von Vorteil, daß die Hybridisierungsreaktion nicht an der Festphase, sondern in Lösung erfolgt. Hybridisierungen in Lösung erfolgen effizienter und erheblich schneller.

Neben den bereits angeführten chemischen Gruppen für die Markierung sind zudem, als bestimmbare Gruppen, enzymatisch aktive Gruppen wie beispielsweise Peroxidase oder β-Galactosidase, fluoreszierende Gruppen wie Fluoreszein oder entsprechende Derivate, Chromophore verschiedenster Art oder lumineszierende Gruppen geeignet. Diese chemischen Gruppen können auf chemischem oder enzymatischem Weg in die Nucleinsäure eingebaut werden. Aber auch Radioisotope, beispielsweise eingebaut in Gegenwart einer terminalen Transferase bzw. T4 RNA-Ligase und eines entsprechend markierten Nukleotids bzw. Oligonukleotids, haben sich als geeignet erwiesen.

Außerdem kann ein Verfahren zum Einführen von nicht-radioaktiv markiertem Desoxynukleotiden in Nukleinsäuren bzw. RNA-Molekülen, die an ihrem 3'-Ende mindestens ein Desoxynukleotid enthalten, das eine nicht-radioaktive Markierungsgruppe trägt, verwendet werden. Ein entsprechendes Verfahren ist in der europäischen Patentanmeldung, Aktenzeichen 95 102 669.9, beschrieben.

Als besonders vorteilhaft hat sich erwiesen, wenn die Markierung der Nukleinsäure bzw. des Polynukleotids mit einem entsprechendem Hapten, wie beispielsweise Biotin oder Digoxigenin, komplexiert in eine Platin-enthaltende Verbindung, wie beispielsweise {Pt(ethylendiamin)(Me₂SO)(hapten-NH(CS)NHCH₃}, durchgeführt wird. Für die Markierung wird eine entsprechend aktivierte Form solcher Platin-Komplexe verwendet. Solche Platin-Verbindungen haben sich als Linkerfunktion als besondere geeignet erwiesen und werden üblicherweise als "Universal Linkage System" (ULS) bezeichnet (EP 0 539 466 / WO 92/01699). Als detektierbare, d.h. als zweite chemische Gruppe haben sich insbesondere Platin-Komplex gekoppelte Gruppen als vorteilhaft erwiesen.

Eine weitere bevorzugte Ausführungsform der Erfindung ist, wenn anstatt einer markierten, komplementären Polynukleotid-Sonde ein Peptid-Nukleinsäure-Derivat mit im wesentlichen zur bestimmenden Sequenz komplementären Basensequenz verwendet wird.

Die Festphase kann prinzipiell aus einer Reihe von Materialien und Formen bestehen, wie z.B. Mikropartikel, sogenannte Beads, porenhaltige oder nicht-permeable Membranen, der inneren Oberflächen von Reaktionsgefäßen wie Teströhrchen oder Mikrotiterplatten. Bevorzugt wird die vorliegende Erfindung an beschichteten Mikrotiterplatten (z.B. Nunclon) durchgeführt, insbesondere solche, bei denen die Beschichtung mit Streptavidin (SA) oder Avidin vorgenommen wurden. Entsprechende Maßnahmen bzw. Festphasen sind dem Fachmann bekannt und beispielsweise in EP 0344578 beschrieben.

Im folgenden werden die einzelnen Verfahrensschritte des erfindungsgemäßen Verfahrens genauer beschrieben:

Die Isolierung von ca. 10 - 20 µg mRNA erfolgt über geeignete Beads entsprechend den Informationen zu dem mRNA-Isolierungskit von Boehringer Mannheim. Die Quantifizierung erfolgt bei OD_{260/280nm}, wobei 2 µg mRNA in 500 µl wässrige Lösung 0,1 OD₂₆₀ₙₘ entsprechen.

Für die Markierung von ca. 10 µg mRNA werden ca. 0,4 µg Biotin-ULS zugegeben und ca. 60 Minuten bei 65°C inkubiert, anschließend mit Ethanol gefüllt und über OD_{260/280nm} quantifiziert.

Für die Hybridisierung werden ca. 100-150 µl/well in einem geeigneten Hybridisierungspuffer vorgelegt und auf ca. 50°C vorgeheizt. Eine DIG-markierte DNA-Probe wird in denaturierter Form zum jeweiligen Reaktionsansatz gegeben, nachdem ca. 50 bis 1000 ng/well der Biotin-markierten mRNA einpipettiert wurden. Als besonders vorteilhafter Hybridisierungspuffer hat sich beispielsweise eine wässrige Lösung erwiesen, die ca. 50% Formamid, 0,1% Laurysarcosin und 0,02% SDS enthält. Die Hybridisierung erfolgt in der Regel zwischen 30 Minuten bis 4 Stunden - die Dauer der Hybridisierung hängt z.B. von der Länge der spezifischen Probesequenz als auch von der Stringenz der Hybridisierungsbedingungen ab - bei ca. 50°C bei 400 rpm. Diese Hybridisierungsbedingungen haben sich überraschenderweise für den spezifischen Nachweis von RNA als gut geeignet erwiesen. Dies ist deshalb überraschend, da auf der einen Seite zwar die erforderliche Stringenz gewährleistet wird, auf der anderen Seite jedoch eine rasche Denaturierung von Protein, wie z.B. einer proteinartigen Beschichtung zu erwarten gewesen wäre.

Von dem Hybridisierungsansatz werden ca. je 100 ml in auf 50°C vorgeheizte SA-beschichtete Mikrotiterplatten-wells pipettiert. Die Inkubation erfolgt bei 50°C/400 rpm in ca. 5 Minuten.

Anschließend wird dekantiert und 3 bis 6 mal bei Raumtemperatur gewaschen. Die anschließende Inkubation mit beispielsweise POD-markiertem 〈DIG〉-Antikörper erfolgt in 30 Minuten bei 37°C und 400 rpm. Die anschließende Detektion erfolgt beispielsweise durch Einpipettieren von Luminol/Iodphenol und Messung nach ca. 3 Minuten.

Erläuterungen zu den Abbildungen:

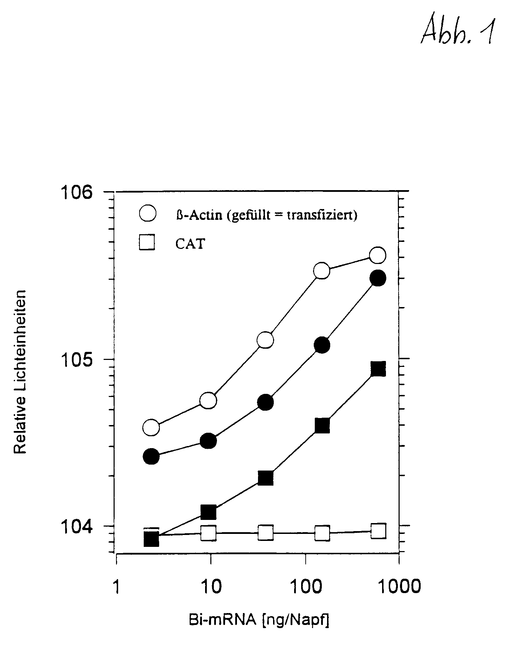

Abbildung 1: Zeigt das Ergebnis von Beispiel 1-4, wobei ○ = β-Actin, ein Gen, welches permanent in Zellen vorkommt, und □ = CAT, ein Gen, welches nicht in eukaryotischen Zellen vorkommt bedeutet; gefüllte Symbole bedeutet "transfiziert".

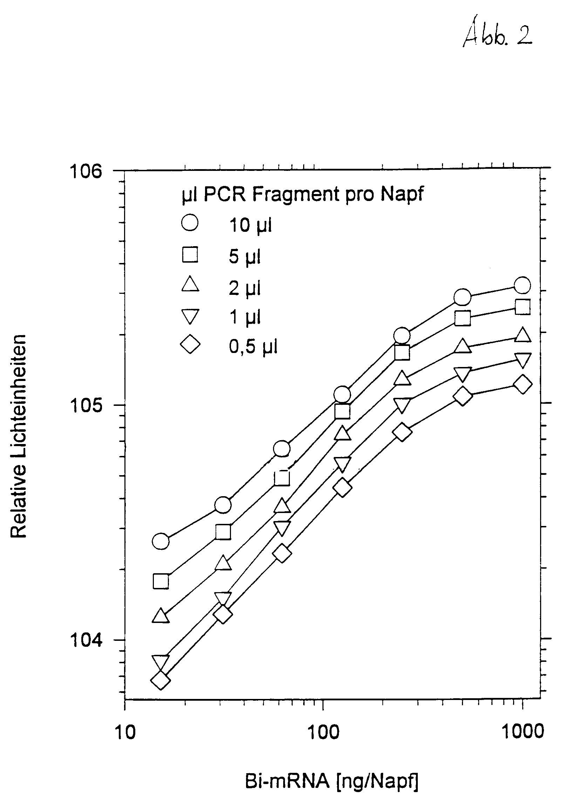

Abbildung 2: Zeigt den Einfluß der Ampliconkonzentration, wobei ○ = 0,5 µl, □ = 1 µl, △ = 2 µl, ∇ = 5 µl und ◇ = 10 µl PCR-Fragment pro well bedeuten.

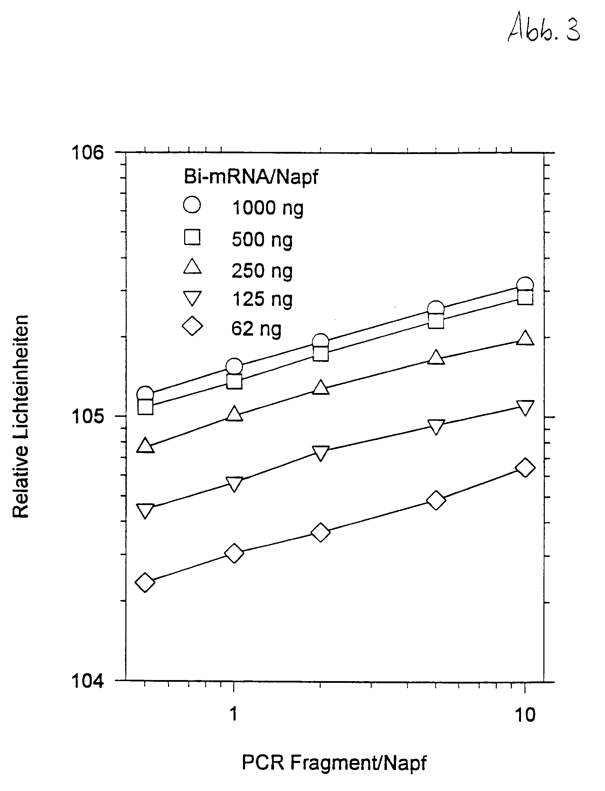

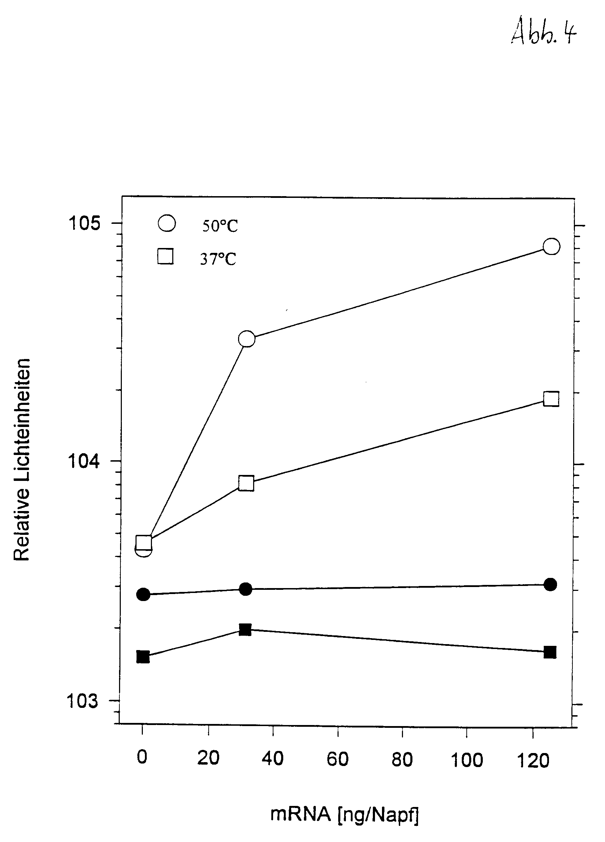

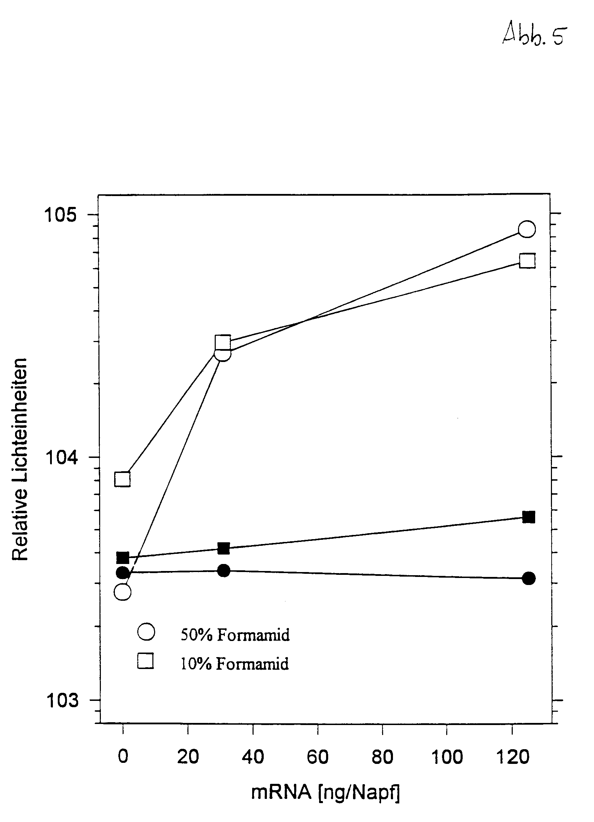

Abbildung 3: Zeigt den Einfluß der Amplificonkonzentration bei konstanter RNA-Konzentration (○ = 1000 ng, □ = 500 ng, △ = 250 ng, ∇ = 125 ng und ◇ = 62 ng Biotin (Bi)-mRNA/well).

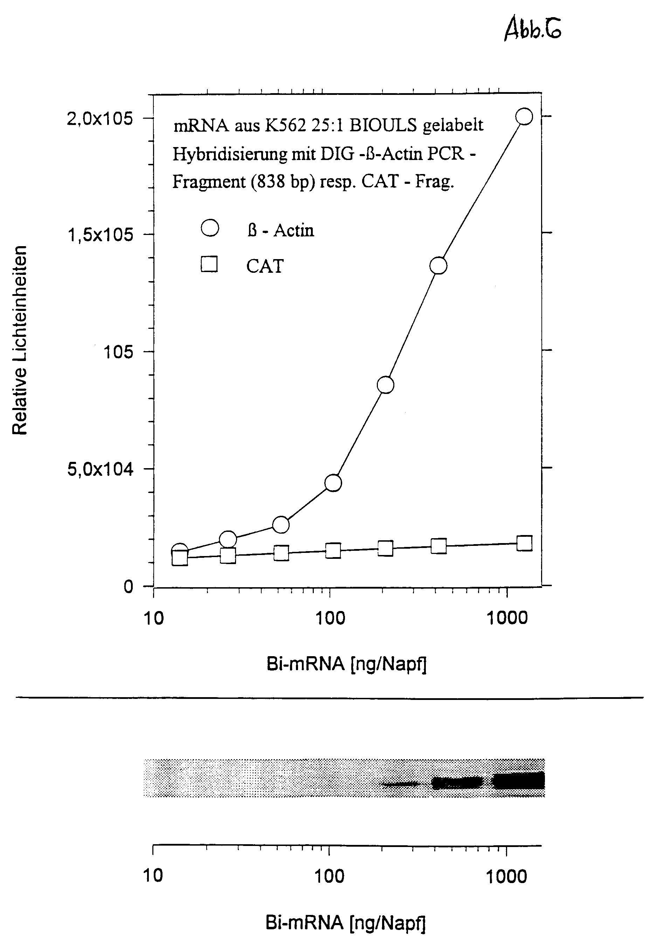

Abbildung 4: Zeigt den Einfluß der Hybridisierungstempeatur, ○ = 50°C und □ = 37°C.

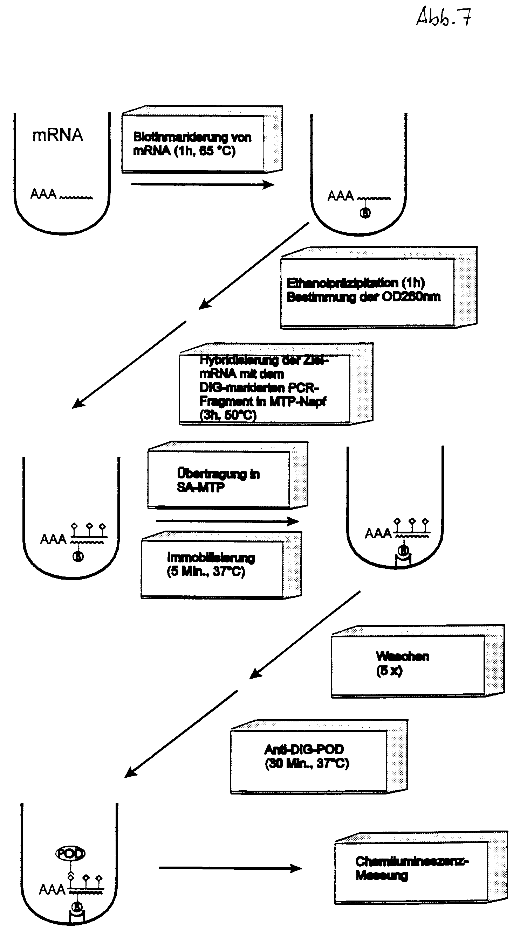

Abbildung 5: Zeigt den Einfluß der Formamid-Konzentration, ○ = 50% Formamid und □ = 10% Formamid.
Abbildung 6: Zeigt einen Vergleich zwischen dem erfindungsgemäßen Northern ELISA- und dem Northern Blot-Verfahren nach dem Stand der Technik, wobei mRNA aus 1 x 562 25:1 BioULS gelabelt ist und die Hybridisierung mit DIG-β-Actin PCR-Fragment (838 bp) bzw. CAT-Fragment durchgeführt wurde; ○ = β-Actin, □ = CAT.
Abbildung 7: Reaktionsschema des erfindungsgemäßen Verfahrens (Northern ELISA).

Die folgenden Beispiele erläutern die Erfindung weiter:

Gesamt mRNA wird über Biotin-Platinkomplexe (Bio-ULS®) mit Biotin markiert. Der Ansatz wird anschließend mit einer für ein Transkript-spezifischen, Digoxigenin (DIG) gelabelten DNA/RNA-Probe hybridisiert. Nach Bindung der mRNAs in einer Streptavidin (SA)-beschichteten Mikrotiterplatte MTP wird die spezifische RNA über 〈DIG〉POD detektiert.
In diesem Bericht wird die Messung von Chloramphenicolacetyltransferase (CAT)-spezifischer mRNA aus mit CAT-Plasmid transfizierten Zellen beschrieben.

### Material und Methoden

Plasmid pSV2CAT wurde erhalten von Dr. Kösters (Universitätsspital Zürich). DNA DIPSTICKs® zur Quantifizierung der mRNA stammen von Invitrogen (USA). Bio-ULS® stammt von Kreatech (Holland). Reagenzien wie Northern Hybridisierungspuffer (50% Formamid, 5 x SSC, 12% Blocking Reagenz in Maleinsäurepuffer, 0,1% Laurylsarcosin, 0,02% SDS), mRNA Isolierungskit, Zellkulturmedien und Transfektionsreagenzien, Reagenzien zur Herstellung der DNA Probes, Streptavidin beschichtete MTP, 〈DIG〉POD, RNase-freier Konjugatverdünnungspuffer (40 mM KPO₄, 1 mM EDTA, 0,25% RSA, pH 6,8) Luminol/Iodphenol als Chemilumineszenzsubstrat und weitere Reagenzien stammen von Boehringer Mannheim. Chemilumineszenzmessungen wurden mit dem Microplate Luminometer LP 96P von Berthold durchgeführt.

### Beispiel 1

### Herstellung der DIG gelabelten Probes

Methoden zur Herstellung geeigneter Probes sind zum Beispiel Amplifikation über PCR, Random Primed Labeling und in vitro Transkription. Die hier verwendeten Probes wurden über geeignete Primer, die innerhalb der codierenden Sequenz der Target-RNA liegen, mittels PCR amplifiziert. Probe-Länge für Actin-Probe: 838 bp; für CAT-Probe: 367 bp (molares Verhältnis im PCR-Mix: dUTP/DIG-dUTP = 3/l). Über Ethidiumbromidfärbung wurde die Ampliconkonzentration abgeschätzt und mit Triethanolamin (TE) pH 8.0 auf ca. 20ng/µl eingestellt.

### Beispiel 2

### Transfektionsansatz

Hela Zellen wurden mittels DOTAP nach Beipackzettel mit pSV2CAT transfiziert. Von den transfizierten Zellen und den unbehandelten Kontrollen wurde die mRNA mittels magnetic beads isoliert.

5 Kulturflaschen mit je 5x10⁶ Zellen/Flaschen (50 ml KM) wurden mit insgesamt 400 µg Plasmid während 6 Stunden transfiziert. 24 Stunden nach der Transfektion wurden die Zellen abtrypsiniert, mit PBS gewaschen und das Pellet in flüssigem Stickstoff gelagert (4,4x10⁶ lebende Zellen, 80% tote Zellen). Analog wurde mit der nicht transfizierten Kontrolle verfahren (1,3x10⁷ lebende Zellen, 5% tote Zellen).

### Beispiel 3

### mRNA Isolierung und Markierung mit Biotin

Die mRNA wurde mittels magnet beads laut Beipackzettel aufgereinigt und die Konzentration über DNA DIPSTICK® bestimmt: Hela (+CAT):
10 µl mit c=360 ng/µl, Hela (-CAT): 23 µl mit c=500 ng/µl. Dann wurde mit Biotin-ULS im Verhältnis RNA/Bio-ULS = 20/l(W/W) 1 Stunde bei 65°C gelabelt, über Ethanolfällung aufgreinigt und in Wasser resupendiert. Anschließende Konzentrationsbestimmung über DNA DIPSTICKS® ergab: Hela (+CAT): 9 µl mit 200 ng/µl, Hela (-CAT): 29 µl mit 80 ng/µl.

### Beispiel 4

### Durchführung Northern ELISA

In eine Zellkultur Rundbodenplatte wurden je 120 µl Northern Hybridisierungspuffer pipettiert und auf 50°C erwärmt. Die DIG markierten Probes aus Beispiel 1 wurden 5 min bei 100°C denaturiert und dann im Eisbad gekühlt. Zu dem Hybridisierungspuffer wurden je 600/150/37,5/9,375 ng Bi-mRNA pipettiert (Verdünnung in TE). Anschließend wurden je 4 µl DIG markierte DNA Probe zupipettiert. Nach 3 Stunden Hybridisierung bei 50°C und 400 rpm wurden je 100µl in eine auf 50°C vorgeheizte tRSA-SA Platte pipettiert und die gemäß Beispiel 3 erhaltene RNA 5 min bei 400 rpm gebunden. Anschließend wurde dekantiert und 5 x mit 0,1 % SSC gewaschen. Einpipettieren von je 100 µl 〈DIG〉POD Konjugat (25 mU/ml) und 30 min Inkubation bei 400 rpm und 37°C. Anschließend wurde dekantiert und 3 x 0,1 SSC gewaschen. Einpipettieren von je 150 µl Luminol/Iodphenol und Messung nach 3 min.

**Tabelle 1**

| Bi-mRNA [ng/well] | Nicht Transfizierte Zellen | | Transfizierte Zellen | |
|---|---|---|---|---|
| | Actin | CAT | Actin | CAT |
| 600,0000 | 411368,0000 | 9254,0000 | 301705,0000 | 86979,0000 |
| 150,0000 | 332754,0000 | 8991,0000 | 120236,0000 | 39765,0000 |
| 37,5000 | 128350,0000 | 9052,0000 | 54657,0000 | 19252,0000 |
| 9,37502, | 56151,0000 | 9001,0000 | 32221,0000 | 12011,0000 |
| 2,3440 | 38877,0000 | 8757,0000 | 26047,0000 | 8331,0000 |

## Patentansprüche

1. Verfahren zur quantitativen Bestimmung einer spezifischen Polynukleotidsequenz in einer Probe, welches folgende Stufen umfaßt:
a) Isolierung der Nukleinsäuren und gegebenenfalls Überführung in einzelsträngige Nukleinsäuren;
b) Markierung der zu bestimmenden einzelsträngigen Nukleinsäure mit einer ersten chemischen, über eine Linkerfunktion gebundenen Gruppe;
c) Hybridisierung der markierten Nukleinsäure mit einer Polynukleotid-Sonde, die mindestens eine einzelsträngige Basensequenz umfaßt, die im wesentlichen komplementär zu der zu bestimmenden Sequenz und mit einer zweiten oder gegebenenfalls weiteren, von der ersten unterschiedlichen chemischen Gruppe markiert ist, in Lösung unter Bedingungen, die für eine Hybridisierung zwischen der zu bestimmenden Sequenz und der komplementären Sondensequenz günstig sind;
d) Immobilisierung des Nukleinsäure-Hybrids an eine Festphase über die erste chemische Gruppe und
e) Detektion der anderen chemischen Gruppe(n).

2. Verfahren nach Anspruch 1, dadurch gekennzeichnet daß mRNA isoliert wird und es sich bei der Polynukleotid-Sonde um ein Oligodesoxyribonukleotid, eine DNS, ein Oligoribonukleotid oder eine RNS handelt.

3. Verfahren nach Anspruch 1, dadurch gekennzeichnet, daß die für die Markierung der Nukleinsäure verwendete erste oder zweite chemische Gruppe ausgewählt werden aus einer enzymatisch aktiven Gruppe, einer fluoreszierenden Gruppe, einem Chromophor, einer lumineszierenden Gruppe, einem spezifisch bindbaren Liganden oder einem Radioisotop.

4. Verfahren nach einem der Ansprüche 1 oder 3, dadurch gekennzeichnet, daß die erste über eine Linkerfunktion gebundene chemische Gruppe Biotin oder ein Biotinderivat darstellt.

5. Verfahren nach Anspruch 1 oder 3, dadurch gekennzeichnet, daß eine Peroxidase, β-Galactosidase, Fluoreszein oder Digoxigen als zweite chemische Gruppe verwendet wird.

6. Verfahren nach einem der Ansprüche 1, 3, 4 oder 5, dadurch gekennzeichnet, daß die Markierung mit einem aktivierten Platinkomplex durchgeführt wird.

7. Verfahren nach Anspruch 1, dadurch gekennzeichnet, daß die erste oder zweite chemische Gruppe auf chemischem oder enzymatischem Weg in die Nukleinsäure eingeführt wird.

8. Verfahren nach Anspruch 7, dadurch gekennzeichnet, daß die enzymatische Markierung mit einer terminalen Transferase oder einer T4 RNA-Ligase und einem durch eine chemische Gruppe markierten Nukleotids oder Olignonukleotid durchgeführt wird.

9. Verfahren nach Anspruch 1, dadurch gekennzeichnet, daß sich bei der markierten, komplementären Polynukleotid-Sonde um ein Peptid-Nukleinsäure-Derivat handelt.

10. Verfahren nach Anspruch 1, dadurch gekennzeichnet, daß eine beschichtete Festphase verwendet wird.

11. Verfahren nach Anspruch 1 oder 10, dadurch gekennzeichnet, daß die Festphase mit Avidin, Streptavidin oder einem entsprechenden Derivat beschichtet ist und die Hybridisierung unter stringenden Bedinungen bei ca. 50°C vorgenommen wird.

12. Verfahren zur quantitativen Bestimmung einer spezifischen Polynukleotidsequenz in einer Probe, welches folgende Stufen umfaßt:
a) Isolierung der Nukleinsäuren und Überführung in einzelsträngige Nukleinsäuren;
b) Auswahl einer Polynukleotid-Sonde, die mindestens eine einzelsträngige Basensequenz umfaßt, die im wesentlichen komplementär zu der zu bestimmenden Sequenz ist;
c) Markierung der Nukleinsäure aus Schritt a) oder des Polynukleotids aus Schritt b) mit einer immobilisierbaren chemischen Gruppe, wobei es sich um über einen Platinkomplex gebundenes Biotin bzw. Biotinderivat handelt;
d) Hybridisierung der Nukleinsäure und des Polynukleotids in Lösung unter Bedingungen, die für eine Hybridisierung zwischen der zu bestimmenden Sequenz und der komplementären Sondensequenz günstig sind;
e) Immobilisierung des Nukleinsäure-Hybrids an eine Festphase über die immobilisierbare chemische Gruppe und
f) Detektion des Hybrids durch einen Antikörper, der spezifisch an DNA/RNA- oder RNA/DNA-Duplexe bindet und durch eine bestimmbare chemische Gruppe markiert ist.

13. Verfahren nach Anspruch 11, dadurch gekennzeichnet, daß es sich bei der isolierten Nukleinsäure um solche mit Poly-dA-Sequenzen handelt.
